Europäisches Patentamt

European Patent Office

Office européen des brevets

(11) Publication number: 0 351 643
A2

# EUROPEAN PATENT APPLICATION

(21) Application number: 89112256.6

(51) Int. Cl.⁴: A61B 5/14

(22) Date of filing: 05.07.89

(30) Priority: 18.07.88 US 220767

(43) Date of publication of application:
24.01.90 Bulletin 90/04

(84) Designated Contracting States:
AT BE CH DE ES FR GB GR IT LI LU NL SE

(71) Applicant: Becton Dickinson and Company
One Becton Drive
Franklin Lakes New Jersey 07417-1880(US)

(72) Inventor: Nugent, Edward L.
Ten Sheffield Road
North Caldwell New Jersey 07006(US)

(74) Representative: Selting, Günther, Dipl.-Ing. et
al
Patentanwälte von Kreisler, Selting, Werner
Deichmannhaus am Hauptbahnhof
D-5000 Köln 1(DE)

(54) Universal safety inoculation devices.

(57) A universal fitting is provided for inoculating sequentially a plurality of examination tubes and/or bottles of a variety of diameters with a single body fluid specimen taken from a patient by a syringe. After taking the sample, the needle on the syringe is discarded and the male luer adapter of the syringe is connected to the female luer on the universal fitting of the invention. The fitting may include a holder arrangement with a skirt for conforming to and frictionally gripping the container receiving the sample. The female luer, in turn, is connected to a needle suspended in the universal fitting of the invention for penetrating the stopper of the container receiving a portion of the sample in the syringe. The arrangement herein may include a rubber sheath valve for covering the needle between successive inoculations. Alternatively, the universal fitting may be connected to a conventional tube holder for introducing the specimen into an evacuated tube, for example.

FIG. 1

## UNIVERSAL SAFETY INOCULATION DEVICES

### Background and Statement of the Invention

Generally speaking, this invention relates to a universal fitting for aiding in the transfer of portions of a body fluid specimen taken by a syringe from a patient so that the potential for a needle stick is reduced substantially during the procedure for inoculating, sequentially, a plurality of specimen blood culture bottles such as a BACTEC[R] Brand Blood Culture Bottle (a product of Becton, Dickinson and Company, Franklin Lakes, New Jersey) from the syringe containing the specimen. Alternatively, the universal device of the invention may be utilized to transfer portions of the body fluid specimen, such as a blood specimen, to a plurality of evacuated tubes for subsequent spin down in a centrifuge to determine the character, presence or absence of a disease in the specimen taken from the patient.

When a blood specimen, for example, is taken from a patient at bedside many times a conventional syringe is used to take, for example, 10 ml of blood from the patient, using a standard veni-puncture technique. This 10 ml of blood will enable the technician to inoculate two blood culture bottles. That is, the blood culture bottles are not manufactured with a controlled amount of vacuum. It is necessary to use, therefore, a proper quantity of a blood specimen, for example, in order to obtain the proper blood to media ratio. That is, the culture bottle will contain a media therein for reacting with the specimen in order to provide certain information to the lab technician or microbiologist. It is important that the proper ratio be maintained in the culture bottle between the specimen portion and the media already in the bottle for reacting with the specimen.

Once the technician takes the blood sample from the patient, the syringe needle is reshielded and/or removed, and a new needle attached to insure sterility during the inoculation procedure of the blood culture bottles. The technician punctures the blood culture bottle stopper and dispenses, for example, 5 ml of the patient's specimen into the bottle. Thereafter, a second blood culture bottle is inoculated, as well, to introduce into the second bottle the second 5 ml of the original specimen taken.

During this transfer procedure, because the needle is contaminated, the technician is subject to needle stick. If, for example, the patient had some contagious disease such as hepatitis or AIDS, the technician is exposed to this disease. Of course, if other diagnostic tests are required, the phlebotomist must perform a second veni-puncture of the patient, and during the subsequent inoculation procedure as discussed above, the technician is again subjected to needle stick of a contaminated needle.

A second procedure involving the subject matter of the invention is for the phlebotomist to collect a patient sample in an evacuated blood specimen tube, such as a VACUTAINER[R] Brand tube. The tube is then labeled and sent to a microbiology laboratory in the hospital where the patient is being treated. The microbiologist uses a needle and syringe to withdraw the specimen from the evacuated tube and, subsequently, to inoculate one or more blood culture tubes or bottles as described above. Again, the microbiologist is exposed to needle prick of a potentially dangerously contaminated needle.

Other procedures of the kind to which the invention here is directed is the inoculation of culture bottles with sputum samples. For example, a sputum sample may be taken from a patient and a saline sputum suspension prepared. Thereafter, the microbiologist uses a needle and syringe to aspirate the sputum suspension and to then inoculate, sequentially, a plurality of culture bottles for obtaining a reaction of the media in the bottle with the sample introduced. It will be appreciated that the microbiologist in this case is subjected to a great deal of exposure of contaminated needles.

With this invention, by contrast, a universal fitting is provided which will accommodate the sequential inoculation procedure as discussed above without any exposure of a needle to the phlebotomist and/or the microbiologist. The arrangement herein is in the form of a structure having on the top surface thereof, a female luer fitting. Connected to the base of the female luer fitting is a needle surrounded by a skirt body structure for the universal fitting of the invention. The needle may or may not have an elastomer sheath valve which is punctured by the needle when an inoculation is made of the culture bottle, but which sheath reseals itself once the needle is removed from the culture bottle to close off the contaminated needle.

Thus, the microbiologist, for example, in the laboratory, takes an evacuated tube, as discussed above, containing a body fluid sample and removes the specimen body fluid sample from the evacuated tube. Thereafter, the needle is removed from the syringe and the universal joint of the invention is utilized. That is, the male luer lock on the syringe is introduced into the female luer lock of the universal fitting of the invention.

With this attachment, the needle for introduction into the culture tube is surrounded by the annular skirt arrangement of the universal fitting of the invention. This skirt arrangement can also be made to have the effect of frictionally engaging the culture bottles to be inoculated. The needle of the universal fitting is introduced into and through the rubber stopper of a culture bottle and a portion of the specimen in the syringe is introduced into the culture bottle. Subsequently, the needle is removed together with the rest of the universal fitting of the invention and placed on an additional culture bottle for a subsequent inocula tion of a second or even third or fourth bottle, as required.

If, on the other hand, the specimen taken from the patient is to be introduced into an evacuated tube for transfer to a lab, the universal fitting of the invention may be connected to a conventional body fluid sample tube holder, such as an evacuated tube holder. The universal fitting of the invention is screwed to the threaded opening in the top of a conventional holder. This has the effect of having the holder surround the needle of the fitting of the invention. Thus, a plurality of evacuated tubes, for example, such as the VACUTAINER[R] Brand evacuated tube may be sequentially inserted into the holder for having the stopper thereof punctured by the needle of the fitting of the invention for introduction of a portion of the sample from the syringe into the tube. This procedure may be repeated as frequently as required in order to introduce portions sequentially of the sample taken from the patient into a plurality of evacuated tubes.

Before describing this invention in more detail, it may be well to note that the fitting of the invention may be comprised of an inexpensive thermoplastic material which may be mass produced from molds in large quantities for single use and disposal so as, again, to reduce the potential for contamination, once use has been made of the fitting of the invention or its exposure to any kind of contaminated body fluid specimen. Such materials include, for example, polystyrene, polypropylene, polyethylene, polyvinyl chloride, polycarbonate, and polyethylene terephthalate.

Other objects and advantages of this invention will be apparent from the following description, the accompanying drawings and the appended claims.

## Description of the Drawings

Fig. 1 is a sectional view in elevation of one embodiment of universal fitting illustrating the invention;

Fig. 2 is a sectional view in elevation of another embodiment illustrating the invention in which a separate fitting arrangement is utilized with a separate container skirt gripping arrangement;

Fig. 3 shows the universal fitting of the embodiment of Fig. 2 separate from the container gripping skirt of the embodiment of Fig. 2;

Fig. 4 shows a conventional evacuated tube holder for inserting sequentially a plurality of evacuated tubes therein for introducing a body fluid sample into the tubes; and

Fig. 5 is a further embodiment of the universal fitting illustrating the invention in the form of a unitary structure including a needle comprised of the same material as the rest of the fitting structure.

## Detailed Description of the Invention

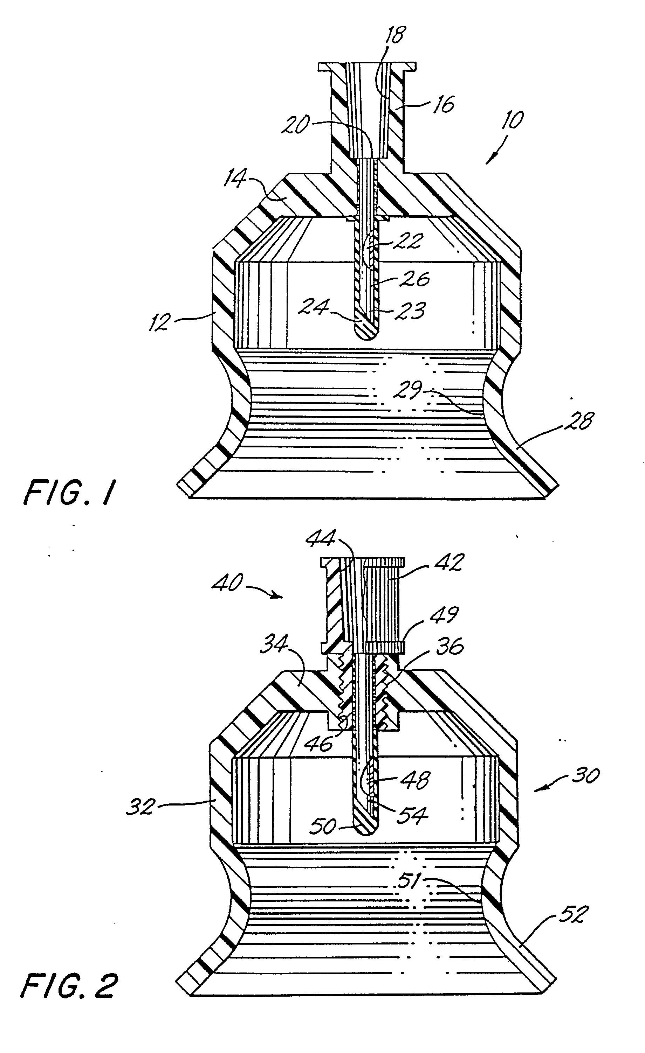

Referring to the drawings in which like reference characters refer to like parts throughout the several views thereof, Fig. 1 shows the uni versal fitting assembly of the invention 10 having an annular body 12 with a top 14 and a depending skirt structure 28 for surrounding and conforming to a culture bottle, for example, when the bottle is receiving an inoculation from a syringe inserted into the structure 10. This conforming to a culture bottle may include the curve 29 in skirt 28.

The fitting 10 includes an integral top 14 having disposed integrally therewith on the top thereof a female luer lock fitting 16, with the internal annular surface 18 thereof for receiving a male luer lock fitting on a syringe. Positioned and affixed or cemented by an adhesive centrally of the top 14 of the universal fitting 10 of the invention is a needle 26, the lumen 22 thereof being in contact at the opening 20 thereof with the base of female luer lock structure 16.

Thus, when a syringe has a needle removed once a body fluid specimen has been taken or aspirated into the syringe, the male luer lock connection of the syringe is inserted into the female structure 16. Thereafter, the needle 26 is inserted through the stopper of a culture bottle. When this happens, the point 23 of needle 26 passes through the stopper of the culture bottle for providing flow communication through the lumen 22 of needle 26 from the interconnection of female luer lock 16 structure with the male luer lock of a syringe. When this penetration procedure is taking place, point 23 of needle 26 also penetrates the sheath valve 24 surrounding needle 26.

Sheath 24 is a conventional elastomeric sheath valve utilized in blood sample applications. The sheath reseals itself once the needle 26 has been removed from the stopper of a culture bottle, in accordance herewith. Thus, the phlebotomist or microbiologist may then move the plunger of the syringe for introducing a portion of the sample in the syringe through the opening 20 into lumen 22 of needle 26, wherein the specimen passes into the blood culture bottle. Subsequently, the syringe with the fitting 10 attached may be removed from the blood culture bottle and transferred to another bottle for a subsequent sequential introduction of a portion of the specimen contained in the syringe.

When this takes place, the resealable sheath 24 recovers the needle 26 until the point 23 of needle 26 is inserted through the stopper of a subsequent culture bottle. During this transfer procedure, the point 23 is surrounded by the annular body 12 so that the technician during such a transfer procedure is not exposed to the contaminated point 23, or its associated sheath 24.

Referring to Fig. 2, in this embodiment, a separate universal connection 40 is shown having a similar female luer lock 42 with the central opening 44 thereof for receiving therein the male luer lock connection of a syringe. In this arrangement, the fitting 40 includes screw threads 36 for cooperating with screw threads 46 on the separate skirt structure 30. Structure 30 includes a top 34 and an integral skirt 32 depending therefrom in the manner of the embodiment in Fig. 1. Skirt 32 may be curved at 51 and 52 to conform to a blood culture bottle in a gripping engagement. The universal fitting 40 of the invention shown in Fig. 2 includes a needle 54 affixed by an adhesive into the universal fitting 40, with a separate sheath valve 50, if required for sequential inoculations. The structure 40 includes a knurled annular grip 49 for screwing the universal fitting 40 into the separate fitting 30. This arrangement, as will be understood, operates in the same manner as the embodiment shown in Fig. 1 described above.

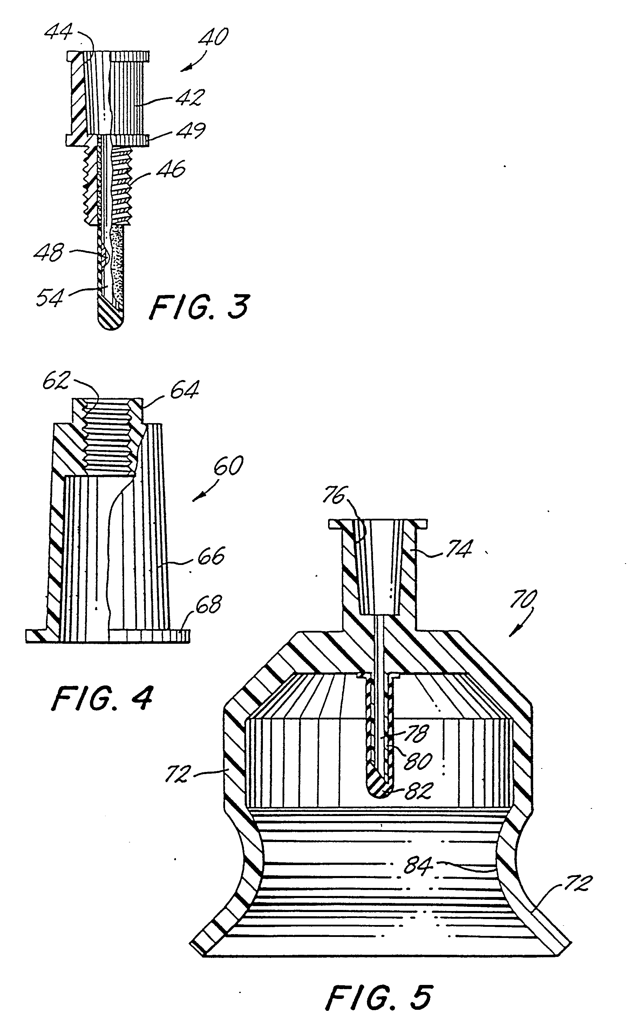

Referring now to Fig. 3, the separate fitting 40 of Fig. 2 is shown separately. As can be seen, the fitting 40 shown in Fig. 3 with the screw threads 46 and the associated needle 54 with its lumen 48 may be utilized with a conventional evacuated blood sample tube holder such as 60 shown in Fig. 4. As can be seen, and as will be appreciated by practitioners-in-the-art, the conventional holder 60 includes a fitting 64 having screw threads 62. Ordinarily, holder 60 would have a double ended needle threaded therein for penetrating the vein of a patient for taking multiple blood samples and introducing them sequentially into evacuated tubes. The holder 60 includes the handle 68 at one end thereof for helping the phlebotomist hold the holder in proper position when taking the single or multiple blood samples from a patient.

As will be appreciated, this very same conventional holder 60 may be utilized with the fitting 40 shown in Fig. 3. That is, the threads 46 cooperate with the threads 62 for introducing and connecting fitting 40 with the holder 60. Thus, a plurality of evacuated tubes may be inserted into holder 60 for sequential introduction of a specimen through lumen 48 of needle 54 for introducing the specimen into the evacuated tubes.

In this connection, the syringe with the needle removed and containing a body fluid sample has the male luer connection thereof introduced into the female luer connection 42 of fitting 40. Thereafter, when the evacuated tube is introduced into holder 60 and the stopper thereof penetrated by needle 54, a portion of the sample in the syringe may be inserted. However, if multiple portions of the sample are to be introduced sequentially, into a plurality of evacuated tubes then needle 54 will have a sheath 50 as shown in Fig. 2 so that the needle is sequentially covered when it is removed from the stopper of an evacuated tube prior to the introduction of needle 52 into the stopper of a subsequent evacuated tube into which a second portion of the specimen in the syringe is to be introduced.

Referring now to Fig. 5, a further embodiment of the invention is shown in the form of structure substantially the same as that shown in Fig. 1. The exception is that the structure 70 shown in Fig. 5 includes an integral needle 80 with a lumen 78 comprised of the same thermoplastic material as the body 72 of the embodiment 70 shown in Fig. 5. Thus, the entire structure shown in Fig. 5, including the annular luer lock connection 74 with its central female luer lock opening 76 for receiving a male luer lock positioned on the front end of a syringe is comprised of the same material, which may be, for example, polycarbonate.

It will be understood that the lower skirt structure 72 of the fitting 70, as well as the embodiments shown in Figs. 1 and 2 are comprised of a flexible thermoplastic material for engaging a culture bottle, for example, in a gripping arrangement for the inoculation thereof. The optional annular curved portion 84 of the skirt 72 helps to provide a conformity to the culture bottle outline as well as a frictional grip thereof. It will be appreciated, that the embodiment shown in Fig. 5, since it can be made in a single molding operation with the needle 80, and without the need for cementing a separate metallic needle into the structure makes the structure 70 of Fig. 5 a particularly desirable structure for mass production of inexpensive throw-away fittings, in accordance herewith.

Thus, as will be appreciated from the above, there is provided in accordance with this invention, a universal throw-away, single-use fitting which enables the microbiologist or phlebotomist or other technician to transfer a body fluid specimen taken from a patient to a plurality of specimen examination culture bottles or evacuated tubes for subsequent testing in the lab. This is achieved, moreover, with a substantial reduction in exposure of these individuals to needle pricks from a contaminated needle. In view of the extraordinary problems involved with contamination since the advent of the AIDS virus and the associated contamination problems with hepatitis, such throw-away devices which provide for only single use and which at the same time simultaneously provide protection from needle prick become very important and useful additions to medical laboratory instrumentation.

While the forms of apparatus herein described constitute preferred embodiments of the invention, it is to be understood that the invention is not limited to these precise forms of apparatus, and that changes may be made therein without departing from the scope of the invention which is defined in the appended claims. For example, whereas one form of body skirt configuration is shown for receiving and conforming to a culture bottle, it will be understood that other configurations may be utilized, as required, depending upon the configuration of culture bottles. For example, the assembly may be arranged to have a different configuration such as square or octagonal, rather than annular depending on the configuration of the container to which it is to be fitted.

## Claims

1. A universal fitting for receiving the male luer connection of a syringe for dispensing portions of a specimen in the syringe sequentially to a plurality of blood culture bottle or evacuated tubes for subsequent examination, the fitting characterized by
    (a) a body portion;
    (b) said body portion having a top surface and a bottom surface;
    (c) a bore in said body portion extending from said top surface to said bottom surface;
    (d) a female luer connection extending from and integral with said top surface;
    (e) a needle in said body portion;
    (f) said needle extending through said bore from said top surface to a point spaced outwardly from said bottom surface;
    (g) said needle having a point at one end thereof;
    (h) a lumen in said needle extending from said needle point to the end of said needle opposite said needle point;
    (i) said lumen, at the end of said needle opposite said needle point, being in fluid flow communication with said female luer connection.
2. The fitting of Claim 1, further characterized by
    (a) a resealable sheath valve extending over said needle point and that portion of said needle extending from said bottom surface.

3. The fitting of Claim 1, further characterized by
    (a) an integral annular skirt extending from said bottom surface and surrounding in spaced relation that portion of said needle extending from said bottom surface.
4. The fitting of Claim 1, further characterized by
    (a) said fitting is comprised of a flexible thermoplastic material whereby said depending annular skirt may grip the outer surface of a culture bottle inserted therein.
5. The fitting of Claim 1, further characterized by
    (a) an integral annular extension extending from said bottom surface;
    (b) said integral annular extension extending along a portion of said needle;
    (c) the outer surface of said integral annular extension having helical threads; and
    (d) whereby said helical threads may be received in a blood sample tube holder.
6. The fitting of Claim 1, further characterized by
    (a) an integral annular extension extending from said bottom surface;
    (b) said integral annular extension extending along a portion of said needle;
    (c) the outer surface of said integral annular extension having helical threads;
    (d) a substantially flat skirt body;
    (e) an annular skirt extending from said skirt body and integral therewith;
    (f) a bore extending centrally through said skirt body; and
    (g) helical screw threads on said skirt body bore for receiving in threaded engagement said helical threads of said integral annular extension.
7. A universal fitting for receiving the male luer connection of a syringe for dispensing portions of a specimen in the syringe sequentially to a plurality of blood culture bottles or evacuated tubes for subsequent examination, the fitting characterized by
    (a) a body portion;
    (b) said body portion having a top surface and a bottom surface;
    (c) a bore in said body portion extending from said top surface to said bottom surface;
    (d) a female luer connection extending from and integral with said top surface;
    (e) a needle in said body portion;
    (f) said needle being integral with said body portion and extending from said bottom surface;
    (g) said needle having a point at the end thereof opposite said bottom surface;
    (h) a lumen in said needle extending from said needle point to and in fluid flow communication with said bore in said body portion;

(i) said bore at said top surface being in fluid flow communication with said female luer connection, and

(j) an integral annular skirt extending from said bottom surface and surrounding in spaced relation said needle depending from said bottom surface.

8. The fitting of Claim 7, further characterized by

(a) a resealable sheath valve extending over said needle point and that portion of said needle extending from said bottom surface.

9. The fitting of Claim 7, further characterized by

(a) said fitting is comprised of a flexible thermoplastic material whereby said depending annular skirt may grip the outer surface of a culture bottle inserted therein.

*FIG. 1*

*FIG. 2*

FIG. 3

FIG. 4

FIG. 5